# EUROPÄISCHE PATENTSCHRIFT

(11) **EP 2 190 359 B1**
(45) Veröffentlichungstag und Bekanntmachung des Hinweises auf die Patenterteilung: **21.01.2015**
(21) Anmeldenummer: 08773627.8
(22) Anmeldetag: 25.06.2008
(51) Int. Cl.: A61B 17/12

(54) **EMBOLISIEREINRICHTUNG**
EMBOLIZATION DEVICE
DISPOSITIF D'EMBOLISATION

(30) Priorität: 14.08.2007 DE 102007038446
(43) Veröffentlichungstag der Anmeldung: 02.06.2010
(73) Patentinhaber: PFM Medical AG, 50996 Köln (DE)
(72) Erfinder: FREUDENTHAL, Franz, La Paz (BO)
(74) Vertreter: Ring & Weisbrodt
(86) Internationale Anmeldenummer: PCT/EP2008/005119
(87) Internationale Veröffentlichungsnummer: WO 2009/021577

(56) Entgegenhaltungen:
- EP-A- 1 738 697
- WO-A-95/25480
- WO-A-96/40024
- WO-A-2006/032291
- WO-A-2007/070792
- JP-A- 8 131 553
- US-A1- 2002 010 481
- US-A1- 2002 128 671
- US-A1- 2005 101 968

## Beschreibung

Die Erfindung betrifft eine Embolisiereinrichtung nach Anspruch 1.

Embolisiereinrichtungen sind in den verschiedensten Formen bekannt. Sie dienen dem Verschluss oder Teilverschluss eines Gefäßes, Organwegs oder einer anderen Körperöffnung. Durch einen solchen Verschluss oder Teilverschluss kann eine künstliche Embolie erzeugt werden, z. B. um innere Blutungen unter Kontrolle zu bringen oder auch zu einem anderen Zweck. Hierzu ist es einerseits bekannt, eine solche Embolisiereinrichtung über einen Ballon-Katheter an der gewünschten Stelle zu expandieren oder alternativ die Embolisiereinrichtung aus einem selbstexpandierenden Material, wie beispielsweise einem Formgedächtnismaterial, herzustellen. Hierbei kann die Embolisiereinrichtung minimal invasiv über einen Katheter zu der gewünschten Stelle im Körper eines Menschen oder Tieres transportiert und dort in der langgestreckten Primärform aus dem Katheter herausgeschoben werden, wodurch die Embolisiereinrichtung ihre voreingeprägte Sekundärform annimmt. Diese kann die unterschiedlichsten Formen aufweisen, wie beispielsweise die einer Spirale oder einer unregelmäßig aufgerollten Raumfigur. In jedem der Fälle ist es ferner grundsätzlich bekannt, thrombogene Fasern an der Embolisiereinrichtung anzubringen, um die Embolisierwirkung zusätzlich zu verstärken.

Hierzu offenbart beispielsweise die EP 0 750 480 B1, dass thrombogene Fasern aus handelsüblichem Z-Twist-Dacron-Fasermaterial in regelmäßigen Abständen über die Länge der Windungen einer Spirale zwischen eng benachbarten Windungen von dieser angeordnet werden. Diese thrombogenen Fasern ragen dann radial zwischen den Windungen der Primärspirale aus dieser hervor. Ein ähnlicher Aufbau ist auch aus der JP-8131553 bekannt. Ebenfalls sieht die JP-2001079011 einen ähnlichen Aufbau vor.

Gemäß der DE 698 31 889 T2 wird eine Embolisiereinrichtung offenbart, bei der ein federnder spulenförmig aufgewickelter Draht auf seiner Außenoberfläche mit Schnitten versehen ist, die einerseits der Verbesserung der Flexibilität des Spulendrahtes dient und andererseits dem Anbringen von thrombogenen Fasern. Die Fasern können in den Draht geknotet, mittels eines Klebers dort befestigt, verschmolzen oder mittels eines anderen Verbindungsverfahrens angebracht werden.

Gemäß der DE 698 26 275 T2 wird eine Embolisiereinrichtung vorgeschlagen, die eine Primärspirale aufweist, die in diverse Sekundärformen umgeformt werden kann. Entlang dieser Primärspirale sind thrombogene Fasern eingeflochten. Diese sind jeweils an einem Ende an einer Windung befestigt und durch einige der dazwischen liegenden Windungen hindurchgefädelt, so dass auf der Außenseite der Primärspirale Schlaufen der thrombogenen Fasern abstehen. Alternativ wird offenbart, eine geflochtene Umhüllung aus einem faserigen Material vorzusehen, das die Primärwände umgibt. Einen ähnlichen Aufbau offenbart auch die DE 698 33 699 T2, bei der ebenfalls thrombogene Fasern durch die Wendel einer Primärspirale bzw. einer Sekundärspirale gefädelt sind. Hierbei stehen dann ebenfalls Schlaufen thrombogener Fasern aus der Spirale hervor. Einen entsprechenden Aufbau zeigt auch die Gefäßverschlusswendel gemäß der DE 698 26 275 T2.

Aus dem Umfang der Wendel bzw. Spirale herausragende Fasern sind nicht nur in der US 6, 187, 027 B1 und der EP 1 584 298 A1, sondern auch in der JP-2005237952 offenbart. Ferner ist ein entsprechender Aufbau auch aus der JP-8131553 und der JP-2001079011 bekannt.

Gemäß der DE 698 31 889 T2 sind entlang der Spirale bzw. von deren Primärmaterial Schlitze vorgesehen, aus denen entsprechende Fasern herausragen.

Eine weitere alternative Lösung zum Anbringen von thrombogenen Fasern an einer Okklusionsspirale ist in der EP 0 778 005 A1 bzw. JP-9276280 offenbart. Hierbei wird eine Vielzahl von Strängen thrombogener Fasern innen durch die Wendel der Okklusionsspirale hindurchgeführt. Die Enden der thrombogenen Faserstränge sind dabei miteinander verbunden.

US 2002/0128671 offenbart eine Embolisiereinrichtung laut dem Oberbegriff des Anspruchs 1.

Die in US 2002/0128671 beschriebenen Embolisiereinrichtungen sind zwar bereits für einen Verschluss einer in einem Körper eines Menschen oder Tieres befindlichen Öffnung besser geeignet als Embolisiereinrichtungen, die keine thrombogenen Fasern aufweisen. Gleichwohl besteht durch die abstehenden thrombogenen Fasern durchaus das Risiko, dass diese sich aus der Embolisiereinrichtung lösen und im Blut an ungewollter Stelle zu Thromben führen können.

Der vorliegenden Erfindung liegt daher die Aufgabe zugrunde, eine Embolisiereinrichtung nach dem Oberbegriff des Anspruchs 1 dahingehend fortzubilden, dass das Risiko, dass sich die thrombogenen Fasern von der Embolisiereinrichtung ungewollt lösen, soweit wie möglich minimiert wird.

Die Aufgabe wird durch eine Embolisiereinrichtung nach Anspruch 1 gelöst. Weiterbildungen der Erfindungen der Erfindung sind in den abhängigen Ansprüchen definiert.

Dadurch wird eine Embolisiereinrichtung geschaffen, bei der aufgrund des Umwickelns des Grundkörpers mit thrombogenen Fasern und deren zusätzliche Befestigung an einer endsertigen Schlaufe eines Innenmandrins diese nicht mehr bzw. im Wesentlichen nicht mehr von diesem abstehen, so dass ein ungewolltes Lösen hier verhindert wird. Aufgrund des Umwickelns des Grundkörpers mit der zumindest einen thrombogenen Faser wird der Grundkörper hinsichtlich seines Durchmessers vergrößert, so dass die thrombogene Wirkung durch Vermindern des Abstands einzelner Wendel einer spiralförmigen Embolisiereinrichtung verbessert werden kann. Ein besonders guter Halt der zumindest einen thrombogenen Faser an dem Grundkörper wird erzielt, wenn dieser nicht nur lediglich drahtförmig, sondern als Primärspirale ausgebildet ist. Hierdurch weist auch der Grundkörper ohne Umwicklung durch zumindest eine thrombogene Faser bereits einen größeren Durchmesser auf, als dies ein nur aus einem Draht gebildeter Grundkörper täte. Nach Umwickeln mit thrombogenen Fasern wird der Durchmesser noch weiter vergrößert und daher die thrombogene Wirkung noch weiter verbessert.

Als für das Umwickeln des Grundkörpers der Emboliereinrichtung besonders vorteilhaft erweist sich das Vorsehen eines Innenmandrins innerhalb des Grundkörpers. Durch einen solchen Innenmandrin wird es deutlich einfacher, an dem Grundkörper anzugreifen, um diesen mit den thrombogenen Fasern umwickeln zu können als ohne einen solchen Innenmandrin, da an diesem endseitig angegriffen und der Grundkörper zum Umwickeln in Längsrichtung gerade gehalten werden kann.

Um einen besonders guten Halt an einem solchen als Primärspirale ausgebildeten Grundkörper für die zumindest eine thrombogene Faser zu ermöglichen, ist diese vorteilhaft außenseitig um die Primärspirale gewickelt. Hierbei bietet es sich besonders vorteilhaft an, ein oder zumindest zwei Faserbündel thrombogener Fasern außenseitig um die Primärspirale herumzuwickeln, da ein besserer Faserverbund bei Vorsehen derartiger Faserbündel vorgesehen und zugleich die Gefahr vermindert werden kann, dass einzelne Fasern von dem Grundkörper abstehen. Ferner kann durch die Verwendung von Faserbündeln der Grundkörper schneller vollständig mit den thrombogenen Fasern umwickelt werden als dies mit lediglich einer einzigen thrombogenen Faser möglich wäre, was sich bei der Herstellung der Embolisiereinrichtung als vorteilhaft erweist. Durch geeignete Wahl der Stärke der Faserbündel sowie der Anzahl der Lagen beim Umwickeln kann der letztendliche Durchmesser des umwickelten Grundkörpers der Embolisiereinrichtung gezielt ausgebildet werden.

Vorteilhaft ist die Primärspirale aus zumindest einem drahtartigen Element ausgebildet, wobei die zumindest eine thrombogene Faser um das zumindest eine drahtartige Element herumgewickelt ist. Bei dieser Ausführungsvariante wird die zumindest eine thrombogene Faser somit nicht erst nach Ausbilden eines Grundkörpers in Form einer Primärspirale, sondern bereits zuvor vor dem Wickeln der Primärspirale zum Erstellen des Grundkörpers um das drahtartige Element für die Primärspirale herumgewickelt. Somit wird die Primärspirale bereits mit einem größeren Durchmesser im Vergleich zu einer normalen Primärspirale ohne derartige thrombogene Fasern ausgebildet. Dieser Grundkörper kann dann in gewünschter Weise zu der Embolisiereinrichtung umgeformt werden. Ein weiteres Umwickeln mit zusätzlichen thrombogenen Fasern kann zwar vorgesehen werden, ist jedoch nicht unbedingt erforderlich. Bei Umwickeln des für die Herstellung des primärspiralenförmigen Grundkörpers verwendeten drahtartigen Elements mit zumindest einer thrombogenen Faser ist die Gefahr eines Ablösens einer solchen thrombogenen Faser noch geringer als bei der zuvor beschriebenen Variante, bei der die Primärspirale des Grundkörpers erst nachfolgend mit zumindest einer thrombogenen Faser umwickelt wird. Der Grund dafür liegt darin, dass bei der Umwicklung des drahtartigen Elements durch die thrombogene Faser diese durch das nachfolgende Aufwickeln der Primärspirale noch fester in diese eingebunden ist, so dass nach dem darauffolgenden Umformen der Primärspirale bzw. des Grundkörpers der Embolisiereinrichtung in die Sekundärformgebung, beispielsweise eine Helix, die zumindest eine thrombogene Faser sich kaum noch aus der Embolisiereinrichtung lösen kann.

Als vorteilhaft erweist es sich, die zumindest eine thrombogene Faser endseitig an dem Grundkörper festzulegen. Hierdurch besteht ebenfalls nicht die Gefahr, dass sich die thrombogene Faser von dem Grundkörper ungewollt wieder löst und ggf. von dem Grundkörper ungewollt abwickelt. Als besonders vorteilhaft erweist es sich hierbei, wenn die zumindest eine thrombogene Faser an einer endseitigen Schlaufe eines Innenmandrins der Primärspirale befestigt, insbesondere dort verknotet ist. Hierbei umgibt die Primärspirale den Innenmandrin, der endseitig zu einer Schlaufe gelegt ist, um ein Angreifen durch ein Platziersystem an der Embolisiereinrichtung zu ermöglichen.

Vorteilhaft kann des weiteren die zumindest eine thrombogene Faser um einen Teil des Innenmandrins, insbesondere eine endseitige Schlaufe des Innenmandrins, der Primärspirale herumgewickelt werden. Hierbei ist es möglich, die zumindest eine thrombogene Faser an der endseitigen Schlaufe des Innenmandrins bzw. an dem Innenmandrin ebenfalls zu befestigen, so dass auch hierdurch wiederum ein ungewolltes Lösen vom Innenmandrin bzw. dem Grundkörper der Embolisiereinrichtung vermieden werden kann.

Als besonders vorteilhaft erweist es sich, eine Embolisiereinrichtung mit einem Grundkörper, wobei der Grundkörper aus einer langgestreckten Primärform in eine Sekundärform überführbar ist, so auszubilden, dass der Grundkörper als Primärspirale mit Innenmandrin ausgebildet ist, wobei der Innenmandrin endseitig unterschiedlich ausgebildete Schlaufen aufweist. Diese endseitig unterschiedlich ausgebildeten Schlaufen können dann zum Angreifen durch ein Platziersystem genutzt werden, so dass sie vorteilhaft an dieses angepasst werden können. Insbesondere kann die proximale Schlaufe zum Befestigen von Haltedrähten und ähnlichen Einrichtungen ausgebildet sein, um diese vor und bei dem Abwurf aus einem Katheter, mittels dessen die Embolisiereinrichtung zu dem Implantationsort transportiert wird, halten und dirigieren zu können. Die distale Schlaufe kann zum Eingriff entsprechend anderer Haltedrähte oder Führungseinrichtungen ausgebildet sein, so dass auch hier ein optimales Angreifen an der Embolisiereinrichtung möglich ist.

Insbesondere kann der Innenmandrin im Bereich der einen endseitigen Schlaufe (der distalen) biegsam, insbesondere als flaches Element ausgebildet. Im Bereich der anderen endseitigen Schlaufe (der proximalen) ist der Innenmandrin hingegen vorteilhaft zum Verbinden mit einem Platziersystem im Wesentlichen biegesteif ausgebildet, insbesondere mit einem runden oder ovalen Materialquerschnitt versehen. Grundsätzlich sind selbstverständlich auch andere Formgebungen möglich, wobei, wie bereits erwähnt, insbesondere eine Anpassung an Halte- und Führungseinrichtungen vorgesehen werden kann, um die Embolisiereinrichtung am Implantationsort möglichst optimal dirigieren und nachfolgend abwerfen zu können. Hierfür eignet sich eine im Wesentlichen biegesteife Ausbildung der Schlaufe, um ein leichtes Dirigieren durch ein Platziersystem zu ermöglichen. Die vorteilhaft als flaches Element ausgebildete distale Schlaufe wird beim Absetzen der Embolisiereinrichtung zuerst aus einem Katheter herausgeschoben und sollte daher möglichst verletzungsfrei an dem Implantationsort austreten. Daher eignet sich hier eine biegsamere Ausbildung als bei der anderen (proximalen) Schlaufe, die zum Dirigieren steifer ausgebildet ist.

Vorteilhaft kann zum Platzieren der Embolisiereinrichtung ein Platziersystem verwendet werden, das zumindest eine Platziereinrichtung mit einem sich durch diese hindurch erstreckenden Haltedraht umfasst, wobei der Haltedraht zum Halten eines proximalen Endes der Embolisiereinrichtung und zum Stabilisieren und Dirigieren der Embolisiereinrichtung beim Vorschieben derselben innerhalb eines Katheters und aus diesem heraus im Wesentlichen biegesteif und zum Abwurf der Embolisiereinrichtung von dem proximalen Ende der Embolisiereinrichtung lösbar ausgebildet ist. Durch ein solches Platziersystem kann die Embolisiereinrichtung besonders gut durch einen Katheter vorgeschoben und an einem Implantationsort abgeworfen werden.

Eine besonders gute Stabilisierung zum Vermeiden eines Knickens der Platziereinrichtung und/oder der Primärspirale der Embolisiereinrichtung kann durch Vorsehen zumindest eines Verbindungsstücks mit einem an der Primärspirale der Embolisiereinrichtung befestigbaren oder an dieser kraftschlüssig angreifbaren Abschnitt, einer mit dem Haltedraht verbindbaren Einrichtung und einem in die Platziereinrichtung eingreifbaren oder an dieser kraftschlüssig angreifbaren Abschnitt vorgesehen werden. Das Verbindungsstück besteht vorteilhaft aus einem für einen Patienten verträglichen Material, insbesondere aus Nitinol oder einem anderen biokompatiblen Material. Das Verbindungsstück kann einen nasenartigen Vorsprung zum Eingreifen in eine Pusherspirale des Platziersystems aufweisen. Hierdurch ist eine besonders gute und sichere Verbindung mit der Pusherspirale möglich.

Vorteilhaft ist die mit dem Haltedraht verbindbare Einrichtung eine Öffnung in dem Verbindungsstück. Durch diese kann der Haltedraht geführt und somit eine Verbindung mit der Primärspirale geschaffen werden. Der Haltedraht umfasst zu diesem Zweck vorteilhaft einen Schlaufenabschnitt und einen länglichen durch den Schlaufenabschnitt fädelbaren Abschnitt. Es kann somit der Schlaufenabschnitt durch die Öffnung in dem Verbindungsstück gefädelt und durch die Schlaufe des Schlaufenabschnitts der längliche Abschnitt gefädelt werden, so dass nach Ziehen an dem Schlaufenabschnitt durch Zusammenwirken mit dem länglichen Abschnitt ein fester Halt den dem Verbindungsstück möglich ist.

Vorteilhaft weist der an der Primärspirale der Embolisiereinrichtung befestigbare Abschnitt ein Außengewinde oder außenseitige Rippen und/oder Nuten zum Eingreifen von Wendeln oder in Zwischenräume zwischen Wendeln der Primärspirale auf. Ein solcher Abschnitt kann problemlos in das proximale Ende der Primärspirale eingeschraubt werden und hält darin fest, so dass eine feste und stabile Verbindung mit der Pusherspirale des Platziersystems erzielt werden kann.

Zum Verbinden der Primärspirale einer Embolisiereinrichtung und dem Platziersystem wird vorteilhaft zunächst ein Verbindungsstück in das proximale Ende der Primärspirale der Embolisiereinrichtung eingeschraubt, werden durch eine Pusherspirale des Platziersystems ein Schlaufenabschnitt und ein länglicher Abschnitt eines Haltedrahtes vorgeschoben, wird der Schlaufenabschnitt durch eine Öffnung in dem Verbindungsstück geschoben, wird durch den Schlaufenabschnitt der längliche Abschnitt hindurch gefädelt, wirkt durch Ausüben einer Zugkraft an dem Schlaufenabschnitt dieser mit dem länglichen Abschnitt zusammen und hält an dem Verbindungsstück fest, werden durch weiteres Ausüben einer Zugkraft Pusherspirale und Primärspirale aufeinander zu gezogen und greift der nasenartige Vorsprung des Verbindungsstücks in das distale Ende der Pusherspirale ein.

Zum Abwerfen der Primärspirale einer Embolisiereinrichtung von einem Platziersystem ist in die Primärspirale und in eine Pusherspirale des Platziersystems eingreifend ein Verbindungsstück vorgesehen und durch ineinander gefädelten Schlaufenabschnitt und länglichen Abschnitt eines sich durch die Pusherspirale hindurch erstreckenden Haltedrahtes gehalten, wobei der Schlaufenabschnitt durch eine Öffnung in dem Verbindungsstück hindurch ragt und durch Zusammenwirken mit dem länglichen Abschnitt an diesem festhält, wird der längliche Abschnitt aus dem Schlaufenabschnitt zurückgezogen, wird der Schlaufenabschnitt aus der Öffnung in dem Verbindungsstück heraus gezogen und wird die Pusherspirale in einen Katheter zurückgezogen und dabei der in die Pusherspirale eingreifende Abschnitt des Verbindungsstücks aus dieser heraus gezogen.

Beim Schieben der Primärspirale der Embolisiereinrichtung mittels einer Pusherspirale als Platziereinrichtung durch einen Katheter hindurch treten große Kräfte auf, die zu einem Knicken der Spiralen führen können. Daher erweist sich das Vorsehen des Verbindungsstücks, das sowohl in die Primärspirale eingreift und in deren Endbereich eine Versteifung herbeiführt als auch dort und seitens der Pusherspirale, an der es ebenfalls angreift, zu einem besseren Kraftübergang führt, als besonders vorteilhaft. Der Haltedraht in Form des Schlaufenabschnitts und des länglichen Abschnitts dient einerseits zum Befestigen der Pusherspirale bzw. der Platziereinrichtung an der Primärspirale, andererseits zum Geradehalten der Verbindung zwischen den beiden Spiralen und natürlich zum gezielten Lösen der Platziereinrichtung von der Embolisiereinrichtung, wenn diese abgeworfen werden soll. Somit erweist sich das Vorsehen eines solchen Verbindungsstücks zwischen einer Primärspirale und einer Platziereinrichtung, wobei es an beiden angreift und eine Kraftübertragung durch Versteifen der Verbindung von beiden erleichtert, sogar unabhängig von der Embolisiereinrichtung gemäß der vorliegenden Erfindung als äußerst vorteilhaft.

Zum Verbinden mit dem Haltedraht kann am proximalen Ende der Embolisiereinrichtung anstelle des Verbindungsstücks jedoch auch eine endseitige Schlaufe vorgesehen sein. Diese kann Teil eines Innenmandrins, wie er vorstehend bereits beschrieben ist, sein oder getrennt von diesem dort vorgesehen werden. Ein Eingreifen des Schlaufenabschnitts und Verbinden mithilfe des länglichen Abschnitts des Haltedrahts ist auch bei Vorsehen einer solchen endseitigen Schlaufe grundsätzlich möglich.

Eine weitere vorteilhafte Ausgestaltung einer Embolisiereinrichtung mit einem Grundkörper, wobei der Grundkörper aus einer langgestreckten Primärform in eine Sekundärform überführbar ist, liegt darin, dass die spiralförmige Sekundärform einen ersten konisch zulaufenden Spiralabschnitt, einen sich an dessen Ende mit geringerem Durchmesser anschließenden, etwa zylindrischen Abschnitt sowie einen von diesem auf der Außenseite des ersten konisch zulaufenden Spiralabschnitts in Richtung zu dessen Ende mit größerem Durchmesser sich erstreckenden, um dieses zumindest teilweise herumgewundenen dritten Abschnitt aufweist. Es wird somit im Prinzip eine zu einem großen Teil doppelt aufeinanderliegende konisch zulaufende Spiralform als Sekundärform der Embolisiereinrichtung gebildet. Der konisch zulaufende Abschnitt der Embolisiereinrichtung ist dabei aus mehreren übereinanderliegenden Grundkörperwindungen gebildet, so dass dieser Abschnitt hierdurch stabilisiert werden kann. Ferner wird eine größere Dichte der einzelnen Windungen des Grundkörpers für die Sekundärform der Embolisiereinrichtung hier geschaffen. Die thrombogene Wirkung der Embolisiereinrichtung kann dadurch optimal erhöht werden.

Bei allen vorstehend genannten Ausführungsformen erweist es sich als vorteilhaft, wenn der Grundkörper aus einem Metall- und/oder Kunststoffmaterial besteht. Insbesondere kann der Grundkörper aus einem Formgedächtnismaterial, insbesondere Nitinol, oder einem anderen Formgedächtnismaterial, bestehen. Für die zumindest eine thrombogene Faser eignet sich besonders die Verwendung von Kunststofffasern oder -Fäden. Besonders vorteilhaft können die Kunststofffasern ausgewählt sein aus der Gruppe enthaltend absorbierende und nicht absorbierende Materialien, natürliche und synthetische Stoffe, insbesondere Polyester, Polyamide, Polypropylen, Polybutylester, expandiertes Polytetrafluorethylen (PTFEe), Polyvinyldifluorethylen (PVDF), Nylon, Leinen, Seide, Katgut.

Der Innenmandrin kann vorteilhaft ein schraubenförmiges oder ein mit außenseitigen Ausbauchungen versehenes Profil aufweisen. Hierdurch passt er optimal in die Primärspirale des Grundkörpers hinein, so dass sich die einzelnen Windungen der Primärspirale auf dem schraubenförmigen oder mit außenseitigen Ausbauchungen versehenen Innenmandrin anlegen können.

Als sehr vorteilhaft erweist es sich ferner, wenn die Primärspirale aus zumindest einem drahtartigen Element gewickelt wird, wobei die Windungen eng benachbart zueinander angeordnet werden. Gerade beim Umwickeln der Primärspirale als Grundkörper der Embolisiereinrichtung mit zumindest einer thrombogenen Faser oder einem thrombogenen Faserbündel kann auf diesem ein besonders guter Halt erzeugt werden, wenn die Windungen der Primärspirale eng benachbart zueinander angeordnet sind. Auch das Umwickeln selbst ist hierbei einfacher möglich als bei Windungen der Primärspirale mit größerem Abstand zueinander. Grundsätzlich ist es natürlich möglich, Primärspiralen zu verwenden, deren Windungen nicht so eng benachbart zueinander angeordnet sind, jedoch wird in den meisten Fällen eine Primärspirale mit eng benachbart zueinander angeordneten Windungen dieser gegenüber bevorzugt.

Zur näheren Erläuterung der Erfindung werden nachfolgend Ausführungsbeispiele von dieser anhand der Zeichnungen näher beschrieben. Diese zeigen in:
- Figur 1: eine perspektivische Ansicht einer ersten Ausführungsform einer erfindungsgemäßen Embolisiereinrichtung in Form einer konisch zulaufenden Embolisierspirale,
- Figur 2: eine Draufsicht auf die Embolisierspirale gemäß Figur 1,
- Figur 3: eine Seitenansicht der Embolisierspirale gemäß Figur 1,
- Figur 4: eine Ansicht von unten der Embolisierspirale gemäß Figur 1,
- Figur 5: eine perspektivische Ansicht der Embolisierspirale gemäß Figur 1 von schräg unten mit endseitig befestigter Platziereinrichtung,
- Figur 6: eine perspektivische Seitenansicht der Embolisierspirale gemäß Figur 5,
- Figur 7: eine perspektivische Ansicht von unten der Embolisierspirale gemäß Figur 5,
- Figur 8: eine Detailansicht eines Grundkörpers einer erfindungsgemäßen Embolisiereinrichtung während des Befestigungsvorgangs thrombogener Fasern an dem Grundkörper,
- Figur 9: eine seitliche Detailansicht des Grundkörpers gemäß Figur 8 in teilweiser Anordnung in einem Katheter und mit teilweise aufgewickelter thrombogener Faser,
- Figur 10: eine vergrößerte Detailansicht eines als Primärspirale ausgebildeten Grundkörpers gemäß Figur 8 mit zwei außenseitig teilweise aufgewickelten thrombogenen Faserbündeln,
- Figur 11: eine Detailansicht eines als Primärspirale ausgebildeten Grundkörpers für eine erfindungsgemäße Embolisiereinrichtung mit endseitiger Schlaufe, die von einem Haltedraht eines Platziersystems ergriffen ist und mit teilweise außenseitig aufgewickeltem Faserbündel,
- Figur 12: eine Detailansicht des Grundkörpers und des Platziersystems gemäß Figur 11, wobei das Faserbündel endseitig im Bereich der Schlaufe an dem Grundkörper befestigt ist,
- Figur 13: eine Detailansicht des Grundkörpers mit endseitiger Schlaufe, in die das Platziersystem eingreift, wobei die thrombogenen Fasern an der Schlaufe durch Umwickeln befestigt sind,
- Figur 14: eine perspektivische Ansicht der Embolisierspirale gemäß Figur 1, befestigt an einem Haltedraht eines Platziersystems,
- Figur 15 - Figur 17: Prinzipskizzen für den Wickelvorgang einer Primärspirale für einen Grundkörper einer erfindungsgemäßen Embolisiereinrichtung mit Innenmandrin mit endseitigen Schlaufen,
- Figur 18: eine Draufsicht auf einen fertiggestellten Grundkörper einer erfindungsgemäßen Embolisiereinrichtung, wobei der Grundkörper einen Innenmandrin mit endseitigen Schlaufen aufweist,
- Figur 19: eine Draufsicht auf die eine endseitige Schlaufe des Innenmandrins, die im Wesentlichen flach ausgebildet ist,
- Figur 20: eine endseitige Detailansicht des Abschnitts des Grundkörpers mit der in Figur 19 in der Draufsicht gezeigten als flaches Element ausgebildeten Schlaufe,
- Figur 21: eine Schnittansicht des Details gemäß Figur 20,
- Figur 22: eine Draufsicht auf die andere endseitige Schlaufe des Innenmandrins gemäß Figur 18, die einen etwa runden Materialquerschnitt aufweist,
- Figur 23: eine Detailansicht des Abschnitts des Grundkörpers mit der in Figur 22 in der Draufsicht gezeigten Schlaufe,
- Figur 24: eine Detail-Schnittansicht des Details gemäß Figur 23,
- Figur 25: eine teilweise geschnittene Draufsicht auf den Grundkörper mit Innenmandrin mit endseitigen Schlaufen gemäß Figur 18,
- Figur 26: eine Draufsicht auf eine aus dem Grundkörper gemäß Figur 18 bzw. 25 gebildete Embolisierspirale als Sekundärform,
- Figur 27: eine Seitenansicht der Embolisierspirale gemäß Figur 26,
- Figur 28: eine Ansicht von unten der Embolisierspirale gemäß Figur 26,
- Figur 29: eine Draufsicht auf die Embolisierspirale gemäß Figur 26, mit detaillierter Darstellung des primärspiralenförmigen Grundkörpers,
- Figur 30: eine Figur 27 entsprechende Seitenansicht der Embolisierspirale gemäß Figur 26, mit detaillierter Darstellung des primärspiralenförmigen Grundkörpers,
- Figur 31: eine Ansicht von unten entsprechend Figur 28 der Embolisierspirale gemäß Figur 26, mit detaillierter Darstellung des primärspiralenförmigen Grundkörpers,
- Figur 32: eine Detailansicht des Eingriffs eines Haltedrahtes in die eine endseitige Schlaufe des Innenmandrins der Embolisierspirale,
- Figur 33: eine seitliche Schnittansicht der Embolisierspirale gemäß Figur 26 in der Darstellung gemäß Figur 27,
- Figur 34: eine Detailansicht der Embolisierspirale gemäß Figur 26 bzw. 29 mit herausgezogenem Innenmandrin, der schraubenförmig ausgebildet ist,
- Figur 35: eine Detailansicht einer Pusherspirale der Platziereinrichtung in langgestreckter Form,
- Figur 36: eine Detailansicht eines Haltedrahts des Platziersystems,
- Figur 37: eine Detailansicht eines Verbindungsstücks zum Einbringen in ein Ende einer Embolisierspirale,
- Figur 38: eine Detailansicht einer zweiten Ausführungsform eines Verbindungsstücks zum Einbringen in eine Embolisierspirale,
- Figur 39: eine vergrößerte Detailansicht des Verbindungsstücks gemäß Figur 38,
- Figur 40: eine Draufsicht auf eine Embolisierspirale mit Verbindungsstück vor dem Einbringen in ein Ende der Embolisierspirale,
- Figur 41: eine Detailansicht des Schlaufenabschnitts des Haltedrahts gemäß Figur 36,
- Figur 42 bis 47: Detailansichten des Ablaufs des Erfassens der Embolisierspirale mit Verbindungsstück gemäß Figur 40 durch den innerhalb der Pusherspirale angeordneten Haltedraht,
- Figur 48 bis 53: Detailansichten eines Lösevorgangs der Embolisierspirale gemäß Figur 40 mit eingebrachtem Verbindungsstück von einer Pusherspirale mit innerhalb von dieser angeordnetem Haltedraht,
- Figur 54: eine Schnittansicht durch ein Detail von durch ein Verbindungsstück und Haltedraht miteinander verbundener Embolisierspirale und Pusherspirale,
- Figur 55: eine Seitenansicht des Details gemäß Figur 54,
- Figur 56: eine Schnittansicht des Details der Verbindung von Verbindungsstück und Haltedraht,
- Figur 57: eine Querschnittsansicht des Details gemäß Figur 56,
- Figur 58: eine Seitenansicht des Details gemäß Figur 56,
- Figur 59: eine Schnittansicht des Details gemäß Figur 54 aus der Rückansicht,
- Figur 60: eine Seitenansicht des Details gemäß Figur 59,
- Figur 61: eine Schnittansicht des Details gemäß Figur 56 aus der Rückansicht,
- Figur 62: eine Schnittansicht des Details gemäß Figur 61, und
- Figur 63: eine Seitenansicht des Details gemäß Figur 61.

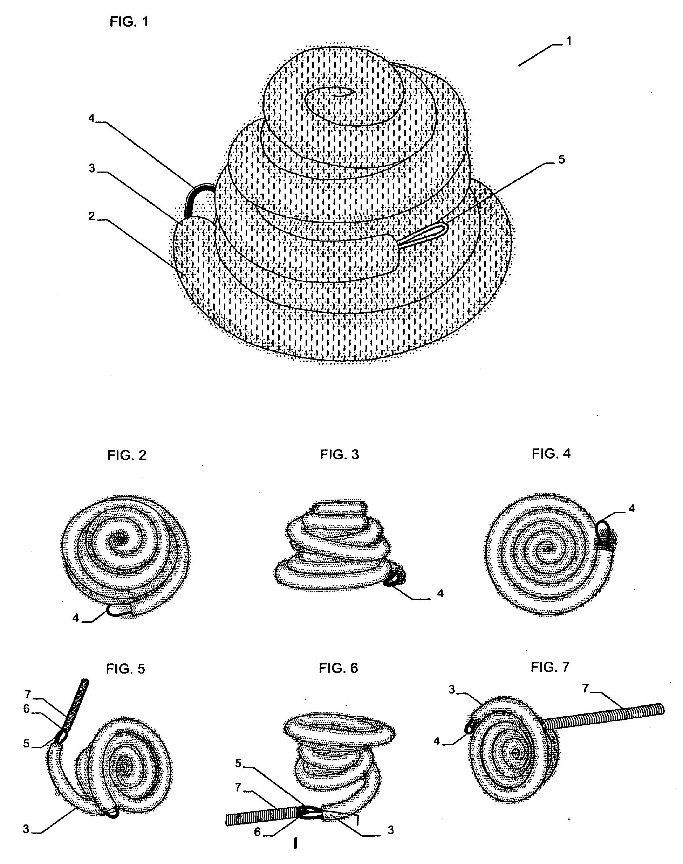

Figur 1 zeigt eine perspektivische Ansicht einer ersten Ausführungsform einer Embolisierspirale 1 mit thrombogenen Fasern 2. Die thrombogenen Fasern 2 sind um einen Grundkörper 3 der Embolisierspirale 1 außenseitig herumgewickelt. Der Grundkörper 3 weist endseitig aus dem umwickelten Abschnitt herausragende Schlaufen 4, 5 auf, die, wie weiter unten erläutert wird, Teil eines Innenmandrins sind. Zumindest die Schlaufe 5 dient zum Befestigen eines Platziersystems, wie in den Figuren 5 bis 7 angedeutet. Hierbei kann ein Haltedraht 6 einer Platziereinrichtung 7 in die Schlaufe 5 eingreifen, um beim Abwurfvorgang einerseits die Embolisierspirale halten und andererseits dirigieren zu können.

Ein Angreifen an der Embolisierspirale ist jedoch auch an der anderen Schlaufe 4 möglich, wobei diese an einem Ende der Embolisierspirale angeordnet ist, wohingegen die Schlaufe 5 auf der Außenseite der Embolisierspirale im Bereich von dessen konisch zulaufenden Abschnitt angeordnet ist.

Wie insbesondere der Draufsicht, der Seitenansicht und der Unteransicht der Embolisierspirale gemäß Figur 2 bis 4 zu entnehmen ist, wird durch das Umwickeln des Grundkörpers mit thrombogenen Fasern ein besonders dichtes Implantat geschaffen, das somit besonders gut zum Hervorrufen eines Thrombus an der gewünschten Stelle im Körper eines Menschen oder Tieres geeignet ist. Wie aus diesen Figuren weiter hervorgeht, ragen hier im Gegensatz zum Stand der Technik keine thrombogenen Fasern aus dem Grundkörper der Embolisierspirale bzw. aus dieser hervor, so dass die Gefahr eines Wanderns derartig im Stand der Technik abstehender thrombogener Fasern durch die Blutbahn eines Menschen oder Tieres nicht mehr oder zumindest kaum noch besteht.

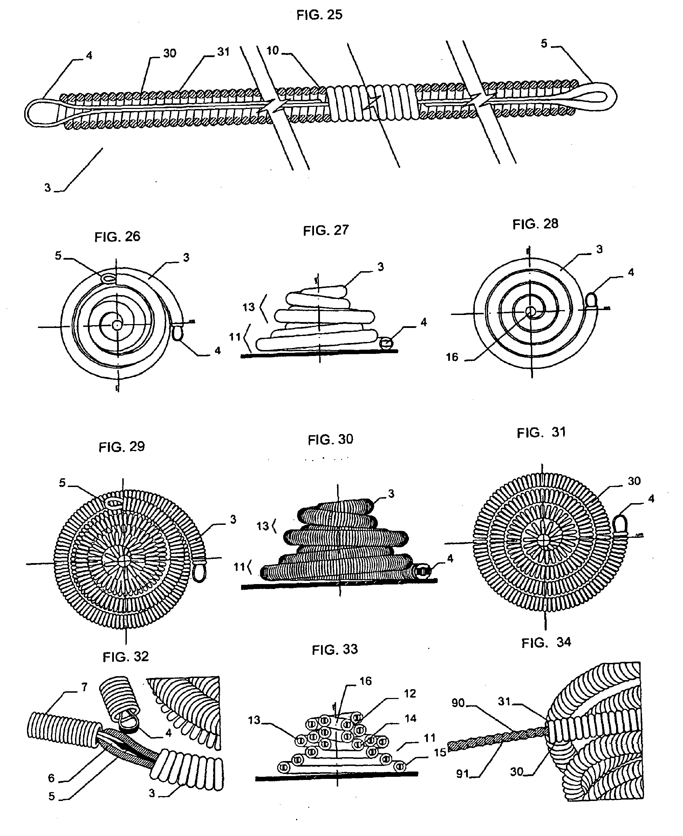

Zusätzlich zu dem Vorsehen thrombogener Fasern wird auch durch die besondere Formgebung der konisch zulaufenden Embolisierspirale, die in einem mittleren Bereich doppelt gelegt ist, ein besonders dichter Körper geschaffen, der den Embolisiervorgang noch weiter unterstützt. Auf diese besondere Formgebung wird weiter unten unter Bezug auf die Figuren 26 bis 33 noch einmal genauer eingegangen.

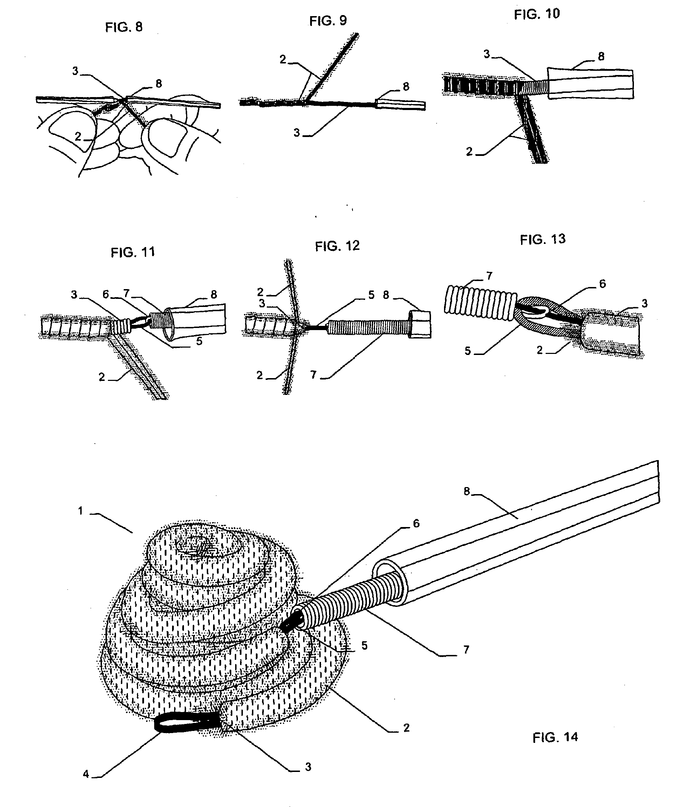

Den Figuren 8 bis 13 kann der Vorgang des Umwickelns des als Primärspirale ausgebildeten Grundkörpers 3 der Embolisierspirale entnommen werden. Figur 8 zeigt dabei das anfängliche Verknoten von Faserbündeln thrombogener Fasern an dem Grundkörper 3. Nach dem endseitigen Befestigen an dem Grundkörper, wie in Figur 8 gezeigt, wird dieser außenseitig mit den Faserbündeln umwickelt, wie in Figur 10 gezeigt. Hierbei können die beiden in Figur 10 gezeigten Faserbündel parallel zueinander gleichsinnig auf der Außenseite um den primärspiralenförmigen Grundkörper 3 herumgewickelt werden. Grundsätzlich ist auch ein gegensinniges Wickeln verschiedener Faserbündel um den Grundkörper herum möglich. In der Darstellung in Figur 9 ist bereits ein Teil des Grundkörpers mit thrombogenen Fasern bzw. Faserbündeln umwickelt. Hierbei kann ein mehrfaches Umwickeln vorgesehen oder auch lediglich ein einlagiges Umwickeln mit entweder nur einem Faserbündel oder mehreren Faserbündeln oder ggf. sogar nur einer thrombogenen Faser, je nachdem, wie die Materialstärke der thrombogenen Faser bzw. thrombogenen Fasern/Faserbündel und der Primärspirale des Grundkörpers gewählt ist. Beispielsweise kann entlang der Längserstreckung des Grundkörpers auch ein unterschiedlicher Wickeldurchmesser der thrombogenen Fasern vorgesehen werden, also teilweise mehrlagig oder mit Fasern größeren Durchmessers eine Umwicklung erfolgen.

In der Darstellung gemäß Figur 11 ist zu erkennen, dass ein breites Faserbündel bis fast an das Ende der Primärspirale des Grundkörpers 3 um diesen herumgewickelt wurde. Aus dem Grundkörper ragt die Schlaufe 5 des Innenmandrins heraus. Diese ist von dem Haltedraht 6 ergriffen, der innerhalb der Platziereinrichtung 7, die ebenfalls spiralförmig ist, angeordnet ist. Die Platziereinrichtung ist innerhalb eines Katheters 8 angeordnet, der in Figur 11 und in Figur 12 ebenfalls angedeutet ist. Der Figur 11 ist ganz deutlich zu entnehmen, dass durch das Umwickeln des Grundkörpers 3 mit den thrombogenen Fasern 2 eine deutliche Querschnittsvergrößerung geschaffen werden kann, was nach dem Umformen des Grundkörpers in die Sekundärform der Embolisierspirale zu der guten thrombogenen Wirkung von dieser führt.

Um die thrombogenen Fasern bzw. Faserbündel 2 an dem Grundkörper 3 befestigen zu können, ist in Figur 12 gezeigt, dass diese endseitig an dem Grundkörper verknotet werden. Selbstverständlich ist auch eine andere Befestigungsart hier möglich, wobei sich durch das Vorsehen der Fasern bzw. Faserbündel ein Verknoten aufgrund der Einfachheit dieses Befestigungsverfahrens natürlich anbietet.

Im Unterschied zu dem Verknoten oder anderweitigen Befestigen der Fasern bzw. Faserbündel 2 am Grundkörper 3 ist in Figur 13 eine Variante gezeigt, bei der die Fasern 2 an der Schlaufe 5 des Innenmandrins befestigt sind. Die Befestigung erfolgt hier durch Umwickeln eines Teilstücks der Schlaufe 5. Nach dem Umwickeln der Schlaufe werden die Fasern 2 beispielsweise ebenfalls verknotet, verschweißt oder auf andere Art und Weise befestigt. Beispielsweise kann das Faserbündel auch wieder ein Stück entlang dem Grundkörper zurückgewickelt, den bereits mit thrombogenen Fasern umwickelten Grundkörper umgebend, und dann dort in geeigneter Weise befestigt werden. Die Art der Befestigung der thrombogenen Fasern bzw. Faserbündel am Grundkörper bzw. dessen Innenmandrin wird vorteilhaft anwendungsspezifisch gewählt, wobei darauf geachtet wird, dass möglichst keine oder kaum abstehende Enden der thrombogenen Fasern verbleiben, die ggf. negative Auswirkungen auf das Embolisierverhalten der Embolisierspirale haben könnten.

Figur 14 zeigt die Embolisierspirale gemäß Figur 1 in Anbringung an der Platziereinrichtung 7 bzw. gehalten von dem Haltedraht 6, wobei die Platziereinrichtung 7 zusammen mit dem Haltedraht 6 innerhalb des Katheters 8 angeordnet ist. Wie auch Figur 14 entnommen werden kann, bildet die Embolisierspirale 1 eine sehr dichte Einheit, die eine besonders gute Embolisierwirkung zeigt.

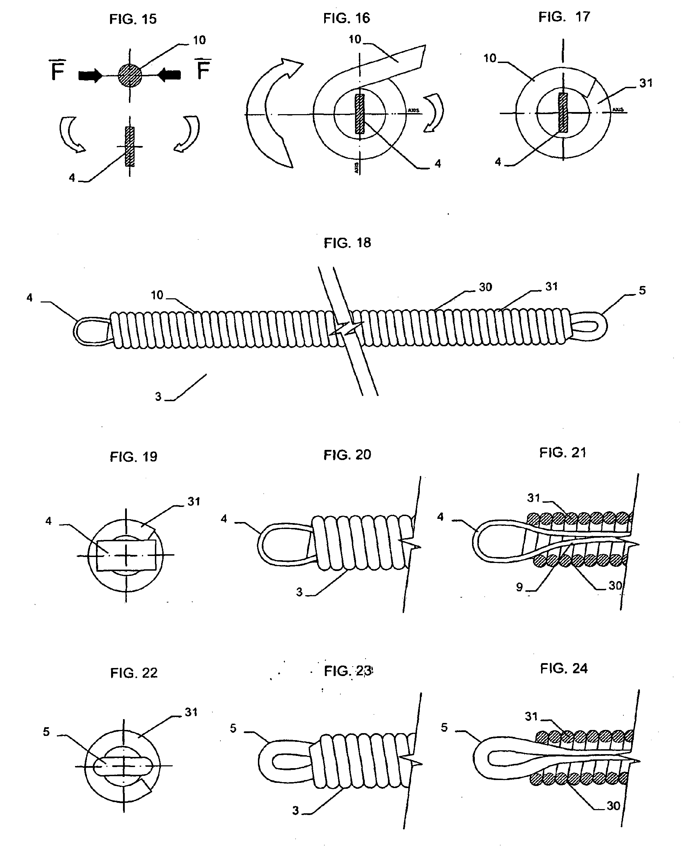

In den Figuren 15 bis 17 ist der Vorgang des Umwickelns des mit endseitigen Schlaufen 4, 5 versehenen Innenmandrins 9 gezeigt. Hierbei wird ein drahtartiges Element 10 um den Innenmandrin 9 herumgewickelt und zwar in der in den Figuren 15 und 16 gezeigten Art und Weise. Hierdurch ergibt sich eine Anzahl von dicht nebeneinander angeordneten Windungen 31, die zusammen die Primärspirale 30 des Grundkörpers 3 bilden. Dies ist der Draufsicht auf den Grundkörper 3 gemäß Figur 18 zu entnehmen. Wie insbesondere der teilweisen Schnittansicht gemäß Figur 25, die ansonsten der Ansicht in Figur 18 entspricht, zu entnehmen ist, erstreckt sich der Innenmandrin 9 im Endbereich bzw. über ein verhältnismäßig großes Teilstück des Grundkörpers hinweg doppellagig, um die Schlaufen 4, 5 zu bilden. Hierdurch wird zugleich dieses Teilstück des Grundkörpers zusätzlich verstärkt. Die Länge dieses doppelt gelegten Teilstücks des Innenmandrins 9 kann somit zum Einstellen der Steifigkeit des Grundkörpers verwendet werden. Soll dieser lediglich im äußersten Endbereich steifer ausgebildet werden, wird nur dort der Innenmandrin doppelt gelegt, wohingegen in dem Falle, in dem der Grundkörper über ein längeres Teilstück hinweg steifer ausgebildet werden soll, der Innenmandrin über ein längeres Teilstück hinweg doppelt gelegt sein kann.

Wie den Detailansichten in den Figuren 19 bis 24 entnommen werden kann, weisen die beiden Schlaufen 4, 5 eine unterschiedliche Formgebung auf. Die Schlaufe 4 ist flacher und breiter ausgebildet als die Schlaufe 5, die einen im Wesentlichen runden Materialquerschnitt aufweist. Wie insbesondere den endseitigen Schnittansichten in den Figuren 21 und 24 entnommen werden kann, ist der Innenmandrin in dem Bereich, in dem die Schlaufe 4 gebildet werden soll bzw. wird, flach und etwas breiter ausgebildet, wohingegen der Innenmandrin zum Ausbilden der Schlaufe 5 in diesem Bereich dicker ausgebildet ist und innerhalb der Primärspirale 30 einen dünneren Querschnitt aufweist, also das Material des Innenmandrins zum Ausbilden der Schlaufe 5 dicker ausgebildet ist, somit einen größeren Materialquerschnitt aufweist. Hierdurch ist ein Angreifen an der Schlaufe 5 sehr gut möglich, da diese eine ausreichende Stabilität zum Angreifen aufweist. Zugleich besteht hier nicht die Gefahr, dass der Haltedraht durch die flache breite Formgebung der Schlaufe 4 abgeknickt werden und sich dadurch beim Zurückziehen in den Katheter zum Abwerfen der Embolisierspirale verhaken kann.

Die Figuren 26 bis 28 bzw. 29 bis 31 zeigen jeweils das Prinzip des Aufwickelns des Grundkörpers 3 zu der Sekundärform der Embolisierspirale 1. Wie der Draufsicht gemäß Figur 26 bzw. 29, jedoch vor allem der Seitenansicht gemäß Figur 27 bzw. 30 und der seitlichen Schnittansicht gemäß Figur 33 entnommen werden kann, wird die spiralförmige Sekundärform der Embolisierspirale zunächst durch Wickeln eines ersten konisch zulaufenden Spiralabschnitts 11 gebildet, an den sich ein zweiter etwa zylindrischer Abschnitt 12 anschließt. Der Anschluss erfolgt an dem Ende 14 des konisch zulaufenden Spiralabschnitts, das einen im Vergleich zum Ende 16 geringeren Durchmesser aufweist. Der zweite zylindrische Abschnitt 12 weist einen etwas geringeren Durchmesser als das Ende 14 des konisch zulaufenden Spiralabschnitts 11 auf. Dies kann besonders gut der Figur 33 entnommen werden. An den zylindrischen Abschnitt 12 schließt sich ein dritter Abschnitt 13 an, der dadurch gebildet ist, dass er in Richtung zu dem Ende 15 des konisch zulaufenden Spiralabschnitts 11 mit größerem Durchmesser auf dessen Außenseite gewunden wird, wobei er den zylindrischen Abschnitt und teilweise ggf. sogar den konisch zulaufenden Spiralabschnitt umgibt. Wie Figur 33 entnommen werden kann, können hier die einzelnen Windungen des dritten Abschnitts 13 auch einanderüberlappen und somit eine besonders dichte Packung an Windungen der Embolisierspirale zur Verfügung stellen. Hierdurch wird nicht nur eine besonders gute thrombogene Wirkung erzielt, sondern auch eine besonders hohe Stabilität in diesem schmaleren Abschnitt der Embolisierspirale. Zugleich kann durch den dritten Abschnitt 13 auch ein besonders guter Halt in der jeweiligen Körperöffnung, die durch die Embolisierspirale verschlossen werden soll, erzielt werden, da durch entsprechende Formgebung des dritten Abschnitts, also Anordnung der Windungen von diesem, bestimmte Bereiche der Embolisierspirale besonders stabil ausgebildet werden können und dabei zumindest einige der Windungen weiter nach außen auskragen können als andere. Wie den Figuren 27, 30 und 33 zu entnehmen, ist dies hier die letzte Windung des Abschnitts 13, die die Schlaufe 5 trägt.

Wie den Ansichten der Embolisierspirale 1 gemäß Figur 28 und 31 entnommen werden kann, die jeweils eine Ansicht von unten dieser Embolisierspiralen zeigen, ist diese von der Unterseite, also beim Blick auf den konisch zulaufenden Spiralabschnitt von unten, regelmäßig als Spirale geformt und mit oder ohne eine zentrale Öffnung 16 versehen, die in Figur 33 und Figur 28 angedeutet ist. Insbesondere nach Umwickeln der Primärspirale 30 mit thrombogenen Fasern oder Faserbündeln, wie in den Figuren 1 bis 14 gezeigt, wird diese innere Öffnung 16 hierdurch verschlossen, so dass die Embolisierwirkung optimiert werden kann. Dies ist insbesondere auch den Figuren 2 und 4 zu entnehmen. Beim Vergleich der Figuren 2 und 29 bzw. 3 und 30 bzw. 4 und 31 ist direkt ersichtlich, dass durch das Umwickeln der Embolisierspirale bzw. von deren Primärspirale 30 mit thrombogenen Fasern 2 eine kompaktere und dichtere Einheit geschaffen werden kann, die eine sehr viel höhere Embolisierwirkung zeigt als die Embolisierspirale ohne derartige thrombogene Fasern.

Figur 32 zeigt eine entsprechende Verbindung zwischen Haltedraht 6 der Platziereinrichtung 7 und Schlaufe 5 des Innenmandrins 9 des Grundkörpers 3 der Embolisierspirale, wie bereits in Figur 13 gezeigt, jedoch hier ohne Umwicklung durch thrombogene Fasern. Das Verbinden des Grundkörpers mit dem Haltedraht bzw. der Platziereinrichtung 7 erfolgt somit ebenso wie bei der Variante, bei der thrombogene Fasern den Grundkörper umgeben. Wie durch den Vergleich der Figuren 13 und 32 klar ersichtlich, entsteht durch das Umwickeln der Enden des Grundkörpers der Embolisierspirale jedoch auch dort eine kompaktere und dichtere Einheit mit zugleich einem größeren Durchmesser, die besser für die Embolisierwirkung genutzt werden kann als die Variante ohne derartige thrombogene Fasern.

Figur 34 zeigt den aus der Primärspirale 30 herausgezogenen Innenmandrin 9, der in dieser Ausführungsvariante schraubenförmig ausgebildet ist, so dass sich die einzelnen Windungen der Primärspirale 30 auf diesem besser abstützen können. Anstelle eines schraubenförmigen Innenmandrins kann beispielsweise auch ein Innenmandrin mit Ausbauchungen auf seiner Außenseite verwendet werden, wobei sich die Windungen der Primärspirale 30 dann in jeweilige Nuten zwischen den Ausbauchungen einfügen können.

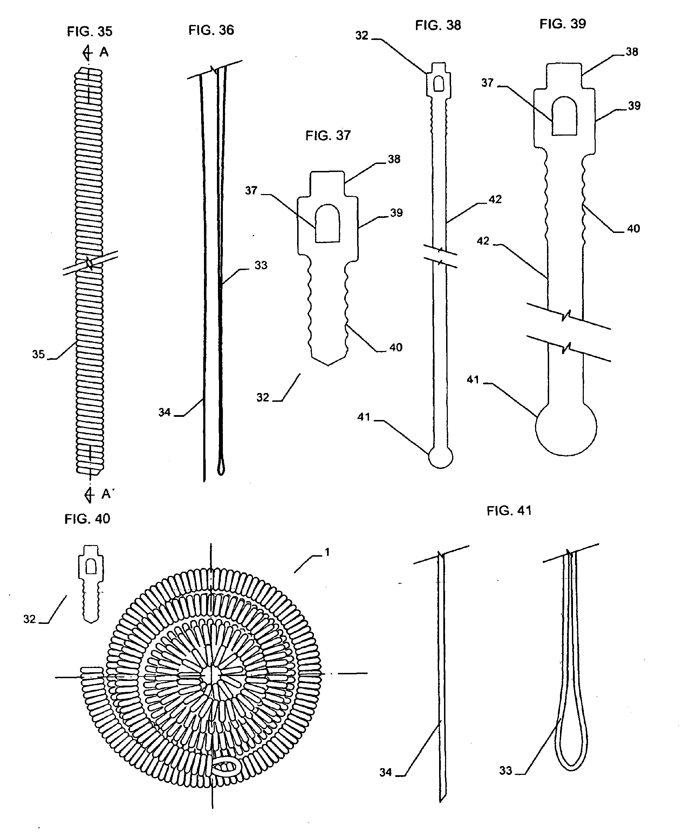

Figur 35 zeigt eine Seitendetailansicht einer Pusherspirale 35 der Platziereinrichtung 7, in die ein Haltedraht mit einem Schlaufenabschnitt 33 und einem länglichen Abschnitt 34 (Figur 36) eingefügt werden kann. Figur 41 zeigt vergrößert das Detail des Schlaufenabschnitts 33 des Haltedrahts. Zum Verbinden mit einer Embolisierspirale sind in den Figuren 37 bis 39 zwei Ausführungsformen eines Verbindungsstücks 32 gezeigt. Beide Ausführungsformen sind flach ausgebildet und weisen einen Kopfteil 39 mit einer zentralen Öffnung 37 und einem nasenartigen Vorsprung 38 und einen Abschnitt 40 mit einer Außenriffelung auf. Die in Figur 37 gezeigte Ausführungsform endet mit einem Abschnitt 40, wohingegen die Ausführungsform nach Figur 38 und 39 im Anschluss an den Abschnitt 40 einen länglichen geraden Abschnitt 42 ohne Außenriffelung und einen punktförmig auskragenden Endabschnitt 41 aufweist. Der längliche gerade Abschnitt 42 kann sich durch eine Primärspirale einer Embolisierspirale hindurch erstrecken, wobei der Endabschnitt 41 dann anstelle der Schlaufe 5 endseitig aus der Primärspirale heraus ragt. Das Verbindungsstück 32 kann aus Nitinol oder einem anderen geeigneten Material bestehen und aus einem Blechstück durch Laserschnitt hergestellt sein.

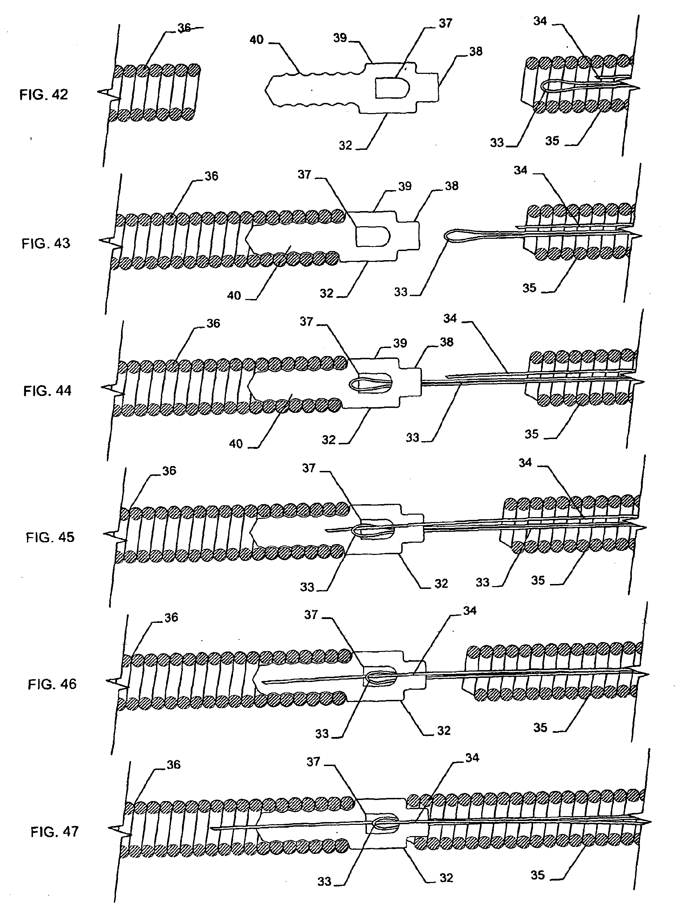

In Figur 40 ist eine Draufsicht auf eine Embolisierspirale 1 in aufgewickelter Form gezeigt, wobei das eine Ende der Primärspirale 36 offen gelassen ist, um darin das Verbindungsstück 32 einzufügen. Der Vorgang des Einfügens des Verbindungsstücks und des Verbindens mit der Pusherspirale 35 ist in den Figuren 42 ff. dargestellt und wird nachfolgend unter Bezug auf diese beschrieben.

Die Figur 42 zeigt ein proximales Ende der Primärspirale 36 der Embolisierspirale, das Verbindungsstück 32, mit dem Abschnitt 40 mit Außenriffelung in Richtung zu der Primärspirale 36 gerichtet, und ein distales Ende der Pusherspirale 35 mit eingefügtem Haltedraht, von dem Schlaufenabschnitt 33 und der längliche Abschnitt 34 gezeigt sind. Im nächsten Schritt des Verbindens von Pusherspirale und Embolisierspirale (Figur 43), um letztere besser durch einen Katheter zum Implantationsort vorschieben zu können, wird das Verbindungsstück mit dem Abschnitt 40 in die Primärspirale 36 eingeschraubt, was aufgrund der Außenriffelung des Abschnitts 40 leicht möglich ist. Dessen Außendurchmesser ist vorteilhaft auf den Innendurchmesser der Primärspirale in diesem Endbereich angepasst. Der Kopfteil 39 liegt außerhalb der Primärspirale 36, der nasenartige Vorsprung 38 ist in Richtung zu der Pusherspirale 35 gerichtet, aus der der Schlaufenabschnitt 33 und der längliche Abschnitt 34 teilweise herausgeschoben sind.

In einem nächsten Schritt, der in Figur 44 dargestellt ist, wird der Schlaufenabschnitt 33 durch die zentrale Öffnung 37 in dem Kopfteil 39 geschoben. Im weiteren Schritt, der in Figur 45 gezeigt ist, wird der längliche gerade Abschnitt 42 durch den Schlaufenabschnitt 33 geschoben. Nachfolgend wird an dem länglichen Abschnitt 34 so lange gezogen (Figur 46), bis der nasenartige Vorsprung 38 in dem distalen Ende der Pusherspirale 35 liegt, was in der Figur 47 gezeigt ist. Durch das Verbindungsstück 32 kann eine kompakte Einheit von Primärspirale und Pusherspirale geschaffen werden, da sich der nasenartige Vorsprung 38 sowohl an der Pusherspirale als auch der Primärspirale abstützt und in diese eingreift. Somit ist die Kraftübertragung von der Pusherspirale auf die Primärspirale beim Vorschieben der Primärspirale in einem Katheter deutlich leichter möglich als ohne das Vorsehen eines solchen Verbindungsstücks 32. Ohne ein solches kann es zu einem Einknicken der Spirale(n) kommen, so dass kein kontrolliertes Vorschieben mehr möglich ist.

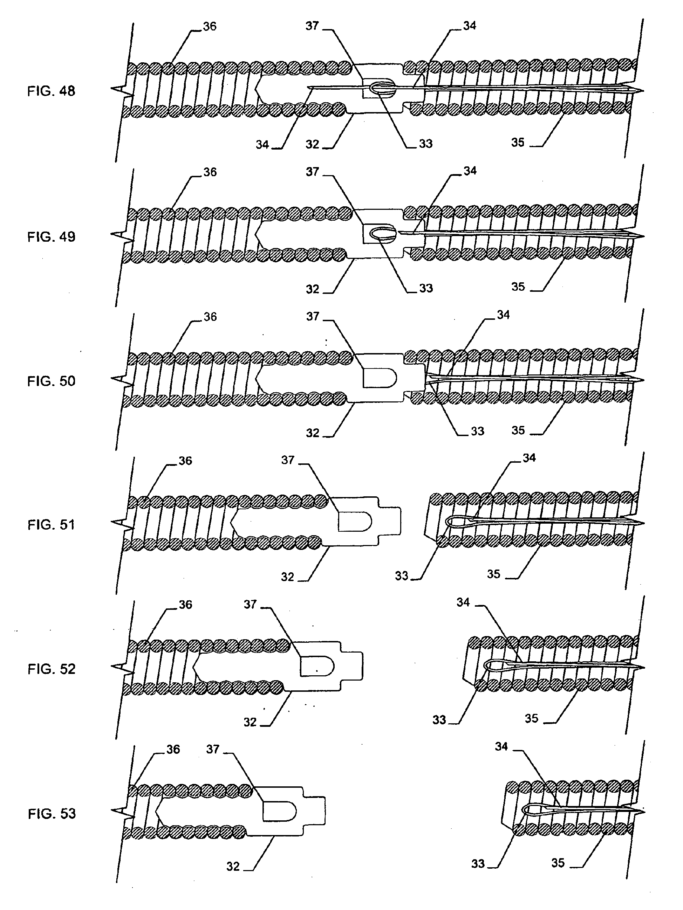

Nach einem Vorschieben der Primärspirale der Embolisierspirale durch den Katheter hindurch an den Implantationsort ist ein Abwerfen der Embolisierspirale dort ebenfalls auf einfache Art und Weise möglich. Wie in den Figuren 48 bis 53 gezeigt, erfolgt das Abwerfen im Prinzip umgekehrt zu den Schritten des Verbindens von Pusherspirale und Primärspirale über das Verbindungsstück 32.

In Figur 48 ist eine ähnliche Situation wie in Figur 47 dargestellt, wobei Pusherspirale 35, Verbindungsstück 32 und Primärspirale 36 unter Zuhilfenahme des Haltedrahts miteinander verbunden sind. Hierbei liegt der nasenartige Vorsprung 38 immer noch in dem distalen Ende der Pusherspirale und der Schlaufenabschnitt 33 des Haltedrahts ist durch die Öffnung 37 des Verbindungsstücks 32 geschoben und hält durch den dort eingefädelten länglichen Abschnitt 34 darin fest. Im nächsten Schritt des Abwerfens, der in Figur 49 gezeigt ist, wurde der längliche Abschnitt 34 aus dem Schlaufenabschnitt 33 zurückgezogen. Im weiteren, in Figur 50 gezeigten Schritt sind der Schlaufenabschnitt 33 aus der zentralen Öffnung 37 und der längliche Abschnitt 34 in die Pusherspirale 35 hinein zurückgezogen. Nachfolgend kann die Pusherspirale in den in Figur 51 nicht dargestellten Katheter wieder zurückgezogen werden, wobei der nasenartige Vorsprung aus dem distalen Ende der Pusherspirale heraus gezogen wird. Pusherspirale und Primärspirale sind somit wieder getrennt. Die Primärspirale kann am Implantationsort sich in ihre eingeprägte Sekundärform verformen, so dass die Embolisierspirale ihre gewünschte Formgebung einnimmt. Die Pusherspirale kann in den Katheter weiter zurückgezogen werden, was in den Figuren 52 und 53 dargestellt ist.

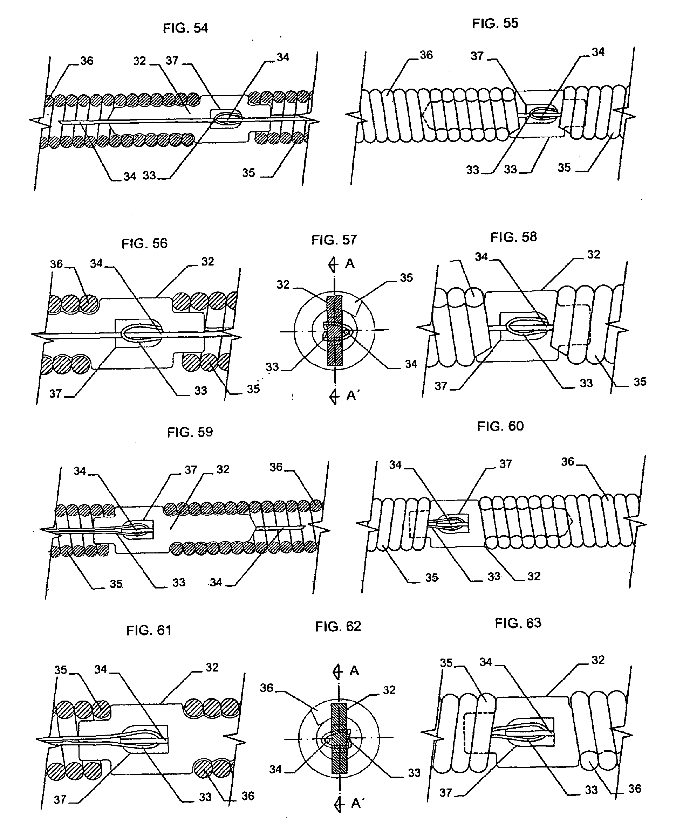

Somit lässt sich die Embolisierspirale auf einfache Art und Weise am Implantationsort abwerfen, wobei ein Wiederverbinden mit dem mit dem Verbindungsstück versehenen Ende der Embolisierspirale ebenfalls grundsätzlich möglich ist, z.B. zum Ändern der Position der Embolisierspirale am Implantationsort oder ggf. auch zum Entfernen der Embolisierspirale von dort. Hierzu wird die Pusherspirale 35 bis zu dem Verbindungsstück 32 insbesondere durch einen Katheter hindurch vorgeschoben, der Schlaufenabschnitt 33 in die zentrale Öffnung 37 in dem Verbindungsstück 32 eingefädelt, der längliche Abschnitt 34 des Haltedrahtes durch die Schlaufe des Schlaufenabschnitts hindurch gefädelt und nachfolgend durch Ziehen an dem Schlaufenabschnitt 33 die Primärspirale an die Pusherspirale herangezogen. Von außerhalb der beiden Spiralen 35, 36 lässt sich lediglich ein Teil des Kopfteils 39 des Verbindungsstücks 32 mit dem in der zentralen Öffnung 37 liegenden Schlaufenabschnitt 33 und durch diesen gefädelten länglichen Abschnitt 34 erkennen, wie dies in der Seitenansicht in Figur 55 zu sehen ist. Figur 54 zeigt eine entsprechende Schnittansicht hierzu, wobei erkennbar ist, dass der längliche Abschnitt 34 über das distale Ende des Verbindungsstücks hinaus in die Primärspirale hinein ragt und dadurch nicht die Gefahr eines Herausrutschens aus dem Schlaufenabschnitt beim Zurückziehen in die Pusherspirale zum Befestigen an dem Verbindungsstück 32 besteht.

Die Figuren 56 bis 58 zeigen noch detaillierter den durch den Schlaufenabschnitt 33 hindurch gefädelten länglichen Abschnitt 34. In den Figuren 59 bis 63 ist dieselbe Verbindung aus der rückwärtigen Ansicht gezeigt, wobei deutlich erkennbar ist, dass der Schlaufenabschnitt 33 aus einem zur Schlaufe gelegten drahtartigen Element gebildet ist. Für das Ausbilden von Schlaufenabschnitt 33 und länglichem Abschnitt 34 kann lediglich ein Haltedraht verwendet werden, der z.B. zweimal mit einer Schlaufe auf sich selbst zurückgelegt ist, so dass ein freies Ende (länglicher Abschnitt 34) und ein Schlaufenabschnitt 33 ebenfalls an diesem Ende entsteht.

Diese Art eines Platzierens bzw. Abwerfens einer Embolisiereinrichtung eignet sich nicht nur bei einer solchen mit Kunststoff-Fäden, sondern auch bei anderen Implantaten, die durch eine Platziereinrichtung durch einen Katheter geschoben können und an denen ein Verbindungsstück angebracht werden kann, das sowohl an dem Implantat als auch an der Platziereinrichtung angreift und durch das eine Stabilisierung der Verbindung von Implantat und Platziereinrichtung und eine bessere Kraftübertragung möglich ist.

Neben den vorstehend beschriebenen und in den Figuren gezeigten Ausführungsformen von Embolisierspiralen sind noch zahlreiche weitere möglich, bei denen jeweils thrombogene Fasern bzw. zumindest eine thrombogene Faser außenseitig um den Grundkörper der Embolisierspirale herumgewickelt sind/ist, wobei der Grundkörper die Form eines drahtartigen Elements und/oder einer Primärspirale und/oder eine andere Formgebung aufweisen kann.

### Bezugszeichenliste:

- 1: Embolisierspirale
- 2: thrombogene Faser
- 3: Grundkörper
- 4: Schlaufe
- 5: Schlaufe
- 6: Haltedraht
- 7: Platziereinrichtung
- 8: Katheter
- 9: Innenmandrin
- 10: drahtartiges Element
- 11: konisch zulaufender Spiralabschnitt
- 12: zylindrischer Abschnitt
- 13: dritter, um den zylindrischen Abschnitt herumgewundener Abschnitt
- 14: Ende von 11
- 15: Ende von 11
- 16: innere Öffnung
- 30: Primärspirale
- 31: Windung
- 32: Verbindungsstück
- 33: Schlaufenabschnitt eines Haltedrahts
- 34: Länglicher Abschnitt eines Haltedrahts
- 35: Pusherspirale
- 36: Primärspirale
- 37: Zentrale Öffnung
- 38: Nasenartiger Vorsprung
- 39: Kopfteil
- 40: Abschnitt mit Außenriffelung
- 41: Endabschnitt
- 42: Länglich gerader Abschnitt
- 90: Ausbauchung
- 91: Nut

## Patentansprüche

1. Embolisiereinrichtung (1) mit einem Grundkörper (3) und mit zumindest einer thrombogenen Faser (2), wobei der Grundkörper (3) aus einer langgestreckten Primärform in eine Sekundärform überführbar ist, wobei die zumindest eine thrombogene Faser (2) um den Grundkörper,(3) herumgewickelt an diesem angeordnet ist, wobei der Grundkörper (3) als Primärspirale (30) ausgebildet ist und wobei die Primärspirale (30) einen Innenmandrin (9) mit zwei endseitigen Schlaufen (4, 5) aufweist,
**dadurch gekennzeichnet, dass**
die zumindest eine thrombogene Faser (2) an einer endseitigen Schlaufe (4, 5) des Innenmandrins (9) der Primärspirale (30) befestigt ist.

2. Embolisiereinrichtung (1) nach Anspruch 1,
**dadurch gekennzeichnet, dass**
die zumindest eine thrombogene Faser (2) oder zumindest zwei Faserbündel thrombogener Fasern (2) außenseitig um die Primärspirale (30) herumgewickelt vorgesehen ist, insbesondere die Primärspirale (30) aus zumindest einem drahtartigen Element (10) ausgebildet ist, wobei die zumindest eine thrombogene Faser (2) um das zumindest eine drahtartige Element (10) herumgewickelt ist und/oder die zumindest eine thrombogene Faser (2) um einen Teil des Innenmandrins (9), insbesondere eine endseitige Schlaufe (4, 5) des Innenmandrins (9), der Primärspirale (30) herumgewickelt ist.

3. Embolisiereinrichtung (1) mit einem Grundkörper (3), wobei der Grundkörper (3) aus einer langgestreckten Primärform in eine Sekundärform überführbar ist, nach einem der vorstehenden Ansprüche,
**dadurch gekennzeichnet, dass**
der Grundkörper (3) als Primärspirale (30) mit Innenmandrin (9) ausgebildet ist, wobei der Innenmandrin (9) endseitig unterschiedlich ausgebildete Schlaufen (4, 5) aufweist, insbesondere der Innenmandrin (9) im Bereich der einen endseitigen Schlaufe (4) biegsam, insbesondere als flaches Element, oder im Bereich der einen endseitigen Schlaufe (5) zum Verbinden mit einem Platziersystem im Wesentlichen biegesteif ausgebildet ist, insbesondere mit einem runden oder ovalen Materialquerschnitt versehen ist.

4. Embolisiereinrichtung (1) mit einem Grundkörper (3), wobei der Grundkörper (3) aus einer langgestreckten Primärform in eine Sekundärform überführbar ist, aus einem Formgedächtnismaterial besteht und mit einem Innenmandrin (9) versehen ist, nach einem der vorstehenden Ansprüche,
**dadurch gekennzeichnet, dass**
die spiralförmige Sekundärform einen ersten konisch zulaufenden Spiralabschnitt (11), einen sich an dessen Ende (14) mit geringerem Durchmesser anschließenden, etwa zylindrischem Abschnitt (12) sowie einen von diesem auf der Außenseite des ersten konisch zulaufenden Spiralabschnitts (11) in Richtung zu dessen Ende mit größerem Durchmesser (15) sich erstreckenden, um diesen zumindest teilweise herumgewundenen dritten Abschnitt (13) aufweist.

5. Embolisiereinrichtung (1) nach einem der vorstehenden Ansprüche,
**dadurch gekennzeichnet, dass**
der Grundkörper (3) aus einem Metall- und/oder Kunststoffmaterial besteht und/oder dass die thrombogenen Fasern (2) Kunststofffasern sind, insbesondere die Kunststofffasern ausgewählt sind aus der Gruppe enthaltend absorbierende und nicht absorbierende Materialien, natürliche und synthetische Stoffe, insbesondere Polyester, Polyamide, Polypropylen, Polybutylester, expandiertes Polytetrafluorethylen (PTFEe), Polyvinyldifluorethylen (PVDF), Nylon, Leinen, Seide, Katgut.

6. Embolisiereinrichtung (1) nach einem der Ansprüche 1 bis 5,
**dadurch gekennzeichnet, dass**
der Innenmandrin (9) ein schraubenförmiges oder ein mit außenseitigen Ausbauchungen (90) und/oder Nuten (91) versehenes Profil aufweist.

7. Verfahren zum Herstellen einer Embolisiereinrichtung (1) nach einem der Ansprüche 1 bis 18,
**dadurch gekennzeichnet, dass**
eine einen Grundkörper (3) der Embolisiereinrichtung (1) bildende Primärspirale (30) mit zumindest einer thrombogenen Faser (2) oder mit zumindest einem Faserbündel mit thrombogenen Fasern (2) umwickelt wird, wobei die zumindest eine thrombogene Faser (2) oder das zumindest eine Faserbündel endseitig an der Primärspirale (30) und/oder an einem Innenmandrin (9) der Primärspirale (30) befestigt wird, und dass die Primärspirale (30) in eine Sekundärspirale umgeformt wird.

8. Verfahren nach Anspruch 7 oder nach dem Oberbegriff des Anspruchs 7,
**dadurch gekennzeichnet, dass**
ein Grundkörper (3) in Form einer Primärspirale (30), der aus einem Formgedächtnismaterial besteht und mit einem Innenmandrin (9) versehen ist, zu einer spiralförmigen Sekundärform gewickelt wird, wobei ein erster konisch zulaufender Spiralabschnitt (11), ein zweiter sich an dessen Ende (14) mit geringerem Durchmesser anschließender etwa zylindrischer Abschnitt (12) und ein dritter Abschnitt (13) gebildet wird, der in Richtung zu dem Ende (15) des Spiralabschnitts *mit* größerem Durchmesser auf dessen Außenseite um diesen zumindest teilweise herumgewunden wird.

9. Verfahren nach einem der Ansprüche 7 oder 8,
**dadurch gekennzeichnet, dass**
die Primärspirale (30) aus zumindest einem drahtartigen Element (10) gewickelt wird, wobei die Windungen (31) eng benachbart zueinander angeordnet werden.

10. Embolisiersystem das eine Embolisiereinrichtung (1) nach einem der Ansprüche 1 bis 6 und ein Platziersystem umfasst
**dadurch gekennzeichnet, dass**
das Platziersystem zumindest eine Platziereinrichtung (7) mit einem sich durch diese hindurch erstreckenden Haltedraht umfasst, wobei der Haltedraht zum Halten eines proximalen Endes der Embolisiereinrichtung (1) und zum Stabilisieren und Dirigieren der Embolisiereinrichtung beim Vorschieben derselben innerhalb eines Katheters (8) und aus diesem heraus im Wesentlichen biegesteif und zum Abwurf der Embolisiereinrichtung (1) von dem proximalen Ende der Embolisiereinrichtung (1) lösbar ausgebildet ist und wobei am proximalen Ende der Embolisiereinrichtung (1) eine endseitige Schlaufe (5) zum Verbinden mit dem Haltedraht (6) vorgesehen ist..

11. Embolisiersystem nach Anspruch 14,
**dadurch gekennzeichnet, dass**
das Platziersystem zumindest ein Verbindungsstück (32) mit einem an der Primärspirale (36) der Embolisiereinrichtung (1) befestigbaren oder an dieser kraftschlüssig angreifbaren Abschnitt (40), einer mit dem Haltedraht verbindbaren Einrichtung (37) und einem in die Platziereinrichtung (36) eingreifbaren oder an dieser kraftschlüssig angreifbaren Abschnitt (38) umfasst, insbesondere das Verbindungsstück (32) einen nasenartigen Vorsprung (38) zum Eingreifen in eine Pusherspirale (36) des Platziersystems aufweist.

12. Embolisiersystem nach Anspruch 10 oder 11,
**dadurch gekennzeichnet, dass**
die mit dem Haltedraht verbindbare Einrichtung eine Öffnung (37) in dem Verbindungsstück (32) ist.

13. Embolisiersystem nach einem der Ansprüche 10 bis 12,
**dadurch gekennzeichnet, dass**
der Haltedraht einen Schlaufenabschnitt (33) und einen länglichen durch den Schlaufenabschnitt (33) fädelbaren Abschnitt (34) umfasst.

14. Embolisiersystem nach einem der Ansprüche 10 bis 13,
**dadurch gekennzeichnet, dass**
der an der Primärspirale (36) der Embolisiereinrichtung (1) befestigbare Abschnitt (40) ein Außengewinde oder außenseitige Rippen und/oder Nuten zum Eingreifen von Wendeln oder in Zwischenräume zwischen Wendeln der Primärspirale (36) aufweist.

15. Verfahren zum Verbinden einer *Primärspirale* (36) einer Embolisiereinrichtung (1) nach einem der Ansprüche 1 bis 6 mit einem Platziersystems nach einem der Ansprüche 10 bis 14,
**dadurch gekennzeichnet, dass**
- ein Verbindungsstück (32) in das proximale Ende der Primärspirale (36) der Embolisiereinrichtung (1) eingeschraubt wird,
- durch eine Pusherspirale (35) des Platziersystems ein Schlaufenabschnitt (33) und ein länglicher Abschnitt (34) eines Haltedrahtes vorgeschoben werden,
- der Schlaufenabschnitt (33) durch eine Öffnung (37) in dem Verbindungsstück (32) geschoben wird,
- durch den Schlaufenabschnitt (33) der längliche Abschnitt (34) hindurch gefädelt wird,
- durch Ausüben einer Zugkraft an dem Schlaufenabschnitt (33) dieser mit dem länglichen Abschnitt (34) zusammenwirkt und an dem Verbindungsstück festhält und weiteres Ausüben einer Zugkraft Pusherspirale und Primärspirale aufeinander zu gezogen werden,
- der nasenartige Vorsprung (38) des Verbindungsstücks (32) in das distale Ende der Pusherspirale (35) eingreift.

## Claims

1. An embolization device (1) comprising a main body (3) and at least one thrombogenic fiber (2), wherein the main body (3) can be converted from an elongated primary form into a secondary form, wherein the at least one thrombogenic fiber (2) is arranged in a configuration wound around the main body (3), wherein the main body (3) is designed as a primary coil (30) and wherein the primary coil (30) comprises an inner mandrel (9) having two end loops (4, 5),
**characterized in that**
the at least one thrombogenic fiber (2) is attached to one end loop (4, 5) of the inner mandrel (9) of the primary coil (30).

2. An embolization device (1) according to claim 1,
**characterized in that**
the at least one thrombogenic fiber (2) or at least two fiber bundles of thrombogenic fibers (2) are provided in a configuration wound around the outside of the primary coil (30), the primary coil (30) is in particular formed by at least one wire-like element (10), wherein the at least one thrombogenic fiber (2) is wound around the at least one wire-like element (10) and/or the at least one thrombogenic fiber (2) is wound around a part of the inner mandrel (9), especially around an end loop (4, 5) of the inner mandrel (9) of the primary coil (30).

3. An embolization device (1) comprising a main body (3), wherein the main body (3) can be converted from an elongated primary form into a secondary form, according to one of the preceding claims,
**characterized in that**
the main body (3) is designed as a primary coil (30) having an inner mandrel (9), wherein the inner mandrel (9) comprises differently shaped loops (4, 5) at the ends, the inner mandrel (9), in the area of one end loop (4), is designed to be flexible, in particular as a flat element, or in the area of one end loop (5) for connection to a positioning system, is substantially stiff, and in particular, is provided with a round or oval material cross section.

4. An embolization device (1) comprising a main body (3), wherein the main body (3) can be converted from an elongated primary form into a secondary form, is made of a shape memory material and is provided with an inner mandrel (9), according to one of the preceding claims,
**characterized in that**
the coil-shaped secondary form comprises a first conically narrowing coil portion (11), an approximately cylindrical portion (12) adjoining the end (14) of lesser diameter of the conically narrowing coil portion (11) and a third portion (13) which starts from the cylindrical portion (11) in the direction of the end (15) of greater diameter thereof and is wound at least partially around same.

5. An embolization device (1) according to one of the preceding claims,
**characterized in that**
the main body (3) is made of a metal and/or plastic material and/or that the thrombogenic fibers (2) are synthetic fibers, the synthetic fibers being in particular selected from the group that comprises absorbent and non-absorbent materials, natural and synthetic fabrics, especially polyester, polyamide, polypropylene, polybutylene ester, expanded polytetrafluoroethylene (PTFEe), polyvinylidene fluoride (PVDF) nylon, cloths, silk, catgut.

6. An embolization device (1) according to one of the claims 1 through 5,
**characterized in that**
the inner mandrel (9) comprises a screw-shaped profile or a profile provided with outer protuberances (90) and/or grooves (91).

7. A method for manufacturing an embolization device (1) according to one of the claims 1 through 6,
**characterized in that**
at least one thrombogenic fiber (2) or at least one bundle of thrombogenic fibers (2) is wound around a primary coil (30) forming a main body (3) of the embolization device (1), wherein the at least one thrombogenic fiber (2) or the at least one bundle of fibers is attached to the end of the primary coil (30) and/or to an inner mandrel (9) of the primary coil, and that the primary coil is converted into a secondary coil.

8. A method according to claim 7 or according to the preamble of claim 7,
**characterized in that**
a main body (3) made of a shape memory material and provided with an inner mandrel (9) in the form of a primary coil (30) is wound to give a coil-shaped secondary form having a first conically narrowing coil portion (11), a second, approximately cylindrical portion (12) that adjoins the end (14) of lesser diameter of the conically narrowing coil portion (11), and a third portion (13) which extends in the direction of the end (15) of greater diameter of the coil portion and is wound at least partially around the outside thereof.

9. A method according to one of the claims 7 or 8,
**characterized in that**
the primary coil (30) is wound from at least one wire-like element (10), wherein the windings (31) are arranged closely adjacent to one another.

10. An embolization system which comprises an embolization device (1) according to one of the claims 1 through 6 and a positioning system,
**characterized in that**
the positioning system comprises at least one positioning device (7) with a retention wire extending through the latter, which retention wire is substantially stiff, in order to hold a proximal end of the embolization device (1) and to stabilize and direct the embolization device as the latter is advanced through the inside of a catheter (8) and out of the latter, and is designed to be detachable from the proximal end of the embolization device (1) in order to release the embolization device (1), and wherein an end loop (5) for the connection with the retention wire (6) is provided at the proximal end of the embolization device (1).

11. An embolization system according to claim 10,
**characterized in that**
the positioning system comprises at least one connection piece (32) with a portion (40) that can be secured on the primary coil (36) of the embolization device (1) or can engage with a force fit thereon, a device (37) connectable to the retention wire, and a portion (38) that can fit into the positioning device (36) or can engage with a force fit thereon, and that in particular the connection piece (32) comprises a nose-like projection (38) for engaging in a pusher coil (36) of the positioning system.

12. An embolization system according to claim 10 or 11.
**characterized in that**
the device connectable to the retention wire is an opening (37) in the connection piece (32).

13. An embolization system according to one of the claims 10 through 12,
**characterized in that**
the retention wire comprises a loop portion (33) and an elongated section (34) which can be threaded through the loop portion (33).

14. An embolization system according to one of the claims 10 through 13,
**characterized in that**
the portion (40) which can be secured on the primary coil (36) of the embolization device (1) comprises an outer thread or outer ribs and/or grooves for the engagement of windings or for engagement in interstices between windings of the primary coil (36).

15. A method for connecting a primary coil (36) of an embolization device (1) according to one of the claims 1 through 6 to a positioning system according to one of the claims 10 through 14,
**characterized in that**
- a connection piece (32) is screwed into the proximal end of the primary coil (36) of the embolization device (1),
- a loop portion (33) and an elongated portion (34) of a support wire are pushed forward by a pusher coil (35) of the placement system,
- the loop portion (33) is pushed through an opening (37) in the connection piece (32),
- the elongated portion (34) is threaded through the loop portion (33),
- by exerting a tensile force on the loop portion (33), the latter interacts with the elongated portion (34) and holds firmly on the connection piece, and by exerting a further tensile force, the pusher coil and the primary coil are drawn toward each other,
- the nose-like projection (38) of the connection piece (32) engages in the distal end of the pusher coil (35).

## Revendications

1. Dispositif d'embolisation (1) comprenant un corps de base (3) et au moins une fibre thrombogénique (2), le corps de base (3) pouvant être transformé d'une forme primaire allongée en une forme secondaire, l'au moins une fibre thrombogénique (2) étant disposée sur le corps de base (3) en étant enroulée autour de celui-ci, le corps de base (3) étant configuré comme spirale primaire (30) et la spirale primaire (30) comprenant un mandrin interne (9) ayant deux boucles (4, 5) aux extrémités,
**caractérisé en ce que**
l'au moins une fibre thrombogénique (2) est fixée à une boucle (4, 5) à l'extrémité du mandrin interne (9) de la spirale primaire (30).

2. Dispositif d'embolisation (1) selon la revendication 1,
**caractérisé en ce que**
l'au moins une fibre thrombogénique (2) ou au moins deux faisceaux de fibres thrombogéniques (2) sont prévus enroulés autour de l'extérieur de la spirale primaire (30), la spirale primaire (30) étant notamment formée par au moins un élément sous forme d'un fil (10), l'au moins une fibre thrombogénique (2) étant enroulée autour de l'au moins un élément sous forme d'un fil (10) et/ou l'au moins une fibre thrombogénique (2) étant enroulée autour d'une partie du mandrin interne (9), notamment autour d'une boucle (4, 5) à l'extrémité du mandrin interne (9) de la spirale primaire (30).

3. Dispositif d'embolisation (1) comprenant un corps de base (3), le corps de base (3) pouvant être transformé d'une forme primaire allongée en une forme secondaire, selon l'une des revendications précédentes,
**caractérisé en ce que**
le corps de base (3) est configuré comme spirale primaire (30) comprenant un mandrin interne (9), le mandrin interne (9) comprenant des boucles (4, 5) différemment configurées aux extrémités, le mandrin interne (9) étant notamment flexible au niveau de l'une boucle d'extrémité (4) et notamment configuré comme une élément plat ou étant essentiellement rigide au niveau de l'une boucle d'extrémité (5) pour être relié à un système de mise en place et étant notamment muni d'une section transversale du matériau ronde ou ovale.

4. Dispositif d'embolisation (1) comprenant un corps de base (3), le corps de base (3) pouvant être transformé d'une forme primaire allongée en une forme secondaire, étant fabriqué en un matériau à mémoire de forme et étant muni d'un mandrin interne (9), selon l'une des revendications précédentes,
**caractérisé en ce que**
la forme secondaire en spirale comprend une première section de spirale taillée en cône (11), une section (12) approximativement cylindrique qui se joint à l'extrémité (14) de la première section ayant un diamètre inférieur ainsi qu'une troisième section (13) qui s'étend à partir de la deuxième section sur l'extérieur de la première section de spirale taillée en cône (11) dans la direction de l'extrémité de celle-ci ayant un diamètre supérieur (15) en étant au moins partiellement enroulée autour de la première section (11).

5. Dispositif d'embolisation (1) selon l'une des revendications précédentes,
**caractérisé en ce que**
le corps de base (3) est fabriqué en métal ou en matière plastique et/ou que les fibres thrombogéniques (2) sont des fibres en plastique, les fibres en plastique étant notamment sélectionnées dans le groupe contenant des matériaux absorbants et non-absorbants, des substances naturelles et synthétiques, notamment des polyesters, des polyamides, du polypropylène, des esters de poylbutyle, du polytétrafluoroéthylène expansé (PTFEe), du polyvinyldifluoroéthylène (PVDF), du nylon, du lin, de la soie, du catgut.

6. Dispositif d'embolisation (1) selon l'une des revendications 1 à 5,
**caractérisé en ce que**
le mandrin interne (9) comprend un profile hélicoïdal ou un profile muni d'évasements extérieurs (90) et/ou de rainures (91).

7. Procédé de fabrication d'un dispositif d'embolisation (1) selon l'une des revendications 1 à 6,
**caractérisé en ce qu'**
au moins une fibre thrombogénique (2) ou au moins un faisceau de fibres thrombogéniques (2) est enroulé autour d'une spirale primaire (30) formant le corps de base (3) du dispositif d'embolisation (1), l'au moins une fibre thrombogénique (2) ou l'au moins un faisceau de fibres étant fixé à l'extrémité de la spirale primaire (30) et/ou à un mandrin interne (9) de la spirale primaire (30), et que la spirale primaire (30) est transformée en une spirale secondaire.

8. Procédé selon la revendication 7 ou selon le préamble de la revendication 7,
**caractérisé en ce qu'**
un corps de base (3) sous forme d'une spirale primaire (30) qui est fabriqué en un matériau à mémoire de forme et muni d'un mandrin interne (9) est enroulé en une forme spirale secondaire, une première section en spirale (11) et taillée en cône, une deuxième section approximativement cylindrique (12) qui se joint à l'extrémité (14) de la première section ayant un diamètre inférieur et une troisième section (13) étant formées, laquelle troisième section est enroulée dans la direction de l'extrémité (15) de la section en spirale ayant le diamètre supérieur sur la face extérieure de cette section et au moins partiellement autour de celle-ci.

9. Procédé selon l'une des revendications 7 ou 8,
**caractérisé en ce que**
la spirale primaire (30) est enroulée à partir d'au moins un élément sous forme d'un fil (10), les spires (31) étant disposées de manière rapprochée les unes par rapport aux autres.

10. Système d'embolisation qui comprend un dispositif d'embolisation (1) selon l'une des revendications 1 à 6 et un système de mise en place,
**caractérisé en ce que**
le système de mise en place comprend au moins un dispositif de mise en place (7) comprenant un fil de maintien qui s'étend à travers celui-ci, le fil de maintien servant à retenir une extrémité proximale du dispositif d'embolisation (1) et à stabiliser et diriger le dispositif d'embolisation pendant le déplacement vers l'avant de celui-ci à l'intérieur d'un cathéter (8) et étant essentiellement rigide pour sortir de celui-ci et pouvant être détaché de l'extrémité proximale du dispositif d'embolisation (1) pour jeter le dispositif d'embolisation (1), et une boucle d'extrémité (5) pour être reliée au fil de maintien (6) étant prévue à l'extrémité proximale du dispositif d'embolisation (1).

11. Système d'embolisation selon la revendication 10,
**caractérisé en ce que**
le système de mise en place comprend au moins une pièce de liaison (32) ayant une section (40) susceptible d'être fixée à la spirale primaire (36) du dispositif d'embolisation (1) ou pouvant venir en prise à force avec celle-ci, un dispositif (37) susceptible d'être relié au fil de maintien et une section (38) qui peut s'engrener avec le dispositif de mise en place (36) ou venir en prise à force avec celle-ci, et que notamment la pièce de liaison (32) comprend une saillie (38) sous forme de nez pour se mettre en prise avec une spirale poussant (36) du système de mise en place.

12. Système d'embolisation selon la revendication 10 ou la revendication 11,
**caractérisé en ce que**
le dispositif susceptible d'être relié au fil de maintien est une ouverture (37) dans la pièce de liaison (32).

13. Système d'embolisation selon l'une des revendications 10 à 12,
**caractérisé en ce que**
le fil de maintien comprend une section de boucle (33) et une section allongée (34) pouvant être passée à travers la section de boucle (33).

14. Système d'embolisation selon l'une des revendications 10 à 13,
**caractérisé en ce que**
la section (40) susceptible d'être fixée à la spirale primaire (36) du dispositif d'embolisation (1) comprend un filetage extérieur ou des nervures extérieures et/ou des rainures pour s'engrener avec des spires ou des interstices entre des spires de la spirale primaire (36).

15. Procédé de liaison d'une spirale primaire d'un dispositif d'embolisation (1) selon l'une des revendications 1 à 6 avec un système de mise en place selon l'une des revendications 10 à 14,
**caractérisé en ce que**
- une pièce de liaison (32) est vissée dans l'extrémité proximale de la spirale primaire (36) du dispositif d'embolisation (1),
- une section de boucle (33) et une section allongée (34) d'un fil de maintien sont poussées vers l'avant par une spirale poussant (35) du système de mise en place,
- la section de boucle (33) est poussée à travers une ouverture (37) dans la pièce de liaison (32),
- la section allongée (34) est passée à travers la section de boucle (33),
- une force de traction est exercée sur la section de boucle (33) ce qui provoque que celle-ci coopère avec la section allongée et adhère à la pièce de liaison et la spirale poussant et la spirale primaire sont tirées l'une vers l'autre en continuant à exercer une force de traction,
- la saillie sous forme de nez (38) de la pièce de liaison (32) vient en prise avec l'extrémité distale de la spirale poussant (35).
